# EUROPEAN PATENT APPLICATION

(11) **EP 4 537 763 A1**
(43) Date of publication of application: **16.04.2025**
(21) Application number: 23203336.5
(22) Date of filing: 12.10.2023
(51) Int. Cl.: A61B 8/00, A61B 5/055

(54) **CONNECTOR SYSTEM**

(71) Applicant: piur imaging GmbH, 1050 Vienna (AT)
(72) Inventor: Bauer, Robert, 82008 Unterhaching (DE); Roth, Robert, 85598 Baldham (DE)
(74) Representative: Zimmermann & Partner Patentanwälte mbB

(57) **Abstract**

The invention relates to a connector system (1) for connecting an add-on unit (5) and a handheld medical imaging device (3), wherein the connector system (1) comprises an add-on unit-side first connector part (10) comprising a recessed receiving portion (20), and a medical-imaging-device-side second connector part (110) comprising a protruding inserter portion (120). The receiving portion (20) comprises an upper rim section (22), a base section (24) and a border section (26) connecting the upper rim section (22) and the base section (24), wherein the border section (26) and the base section (24) enclose an obtuse angle (α). The inserter portion (120) comprises a bottom section (124) and an edge section (126) connected to the bottom section (124). The edge section (126) is form-fit to the border section (26) such that the inserter portion (120) is receivable by the receiving portion (20) in a unique pre-determined orientation with respect to each other. The receiving portion (20) and the inserter portion (120) each comprises a magnetic element (50) configured to provide a magnetic connection between each other to fixate the inserter portion (120) in the receiving portion (20) in the pre-determined orientation.

## Description

The present application relates to a connector system for a hand-held medical imaging device.

Hand-held medical devices can be used for diagnostic purposes as well as therapeutic purposes. Well-known examples of hand-held medical imaging devices are ultrasonic devices (ultrasound, ultrasound probe).

For various applications, a precise tracking of the hand-held medical imaging device is necessary, e.g. for providing a 3D ultrasound image.

For example, tracking can comprise the determination of an absolute position of the hand-held medical imaging device in a space. In an example, the tracking can comprise the determination of a positional change of the hand-held medical imaging device (determination of the relative position). The positional change can comprise a movement of the hand-held medical imaging device from a first location to a second location and/or a rotation of the hand-held medical imaging device and/or a tilt of the hand-held medical imaging device. The tilt can be a roll. The tilt can be a yaw. The tilt can be a pitch.

Tracking can be performed with help of a tracking target mounted at the hand-held medical imaging device. The tracking target can be an active tracking target. The tracking target can be a passive tracking target. Tracking can be performed with help of a sensor or a tracking device arranged at the hand-held medical imaging device.

An easy and safe arrangement of such a tracking target, sensor and/or tracking device is desirable to improve the tracking. Hence, there is a need for a reliable connection means for the connection between the hand-held medical imaging device and the add-on tracking device, sensor, tracking target and/or optical target.

This problem is solved by the connector system according to claim 1, the first connector part according to claim 13, the second connector part according to claim 14, and the method according to claim 12. The problem is also solved by the medical system according to claim 15.

A first aspect of the invention is related to a connector system for connecting an add-on unit and a hand-held medical imaging device. The connector system comprises a add-on-unit-side first connector part comprising a recessed receiving portion and a medical-imaging-device-side second connector part comprising a protruding inserter portion. The receiving portion comprises an upper rim section, a base section and a border section connecting the upper rim section and the base section, wherein the border section and the base section enclose an obtuse angle. The inserter portion comprises a bottom section and an edge section connected to the bottom section. The edge section is form-fit to the border section such that the inserter portion is receivable by the receiving portion in a unique pre-determined orientation with respect to each other. The receiving portion and the inserter portion each comprises a magnetic element configured to provide a magnetic connection between each other to fixate the inserter portion in the receiving portion in the pre-determined orientation.

The receiving portion can be configured for reversible, repeated receiving of the inserter portion in the unique pre-determined orientation. The inserter portion can be configured for reversible, repeated insertion in the receiving portion in the unique pre-determined orientation.

Particularly, a small, compact connector system can be provided which can comprise a repeatedly releasable connection. Advantageously, the connector system can be reusable. The receiving portion can be reusable. The inserter portion can be reusable.

The upper rim section can fully surround the base section. In an embodiment, the upper rim section surrounds a portion of the base section along the circumference of the base section. In an embodiment, the upper rim section surrounds the base section along more than 30% of the circumference of the base section. In an embodiment, the upper rim section surrounds the base section along more than 50% of the circumference of the base section. In an embodiment, the upper rim section surrounds the base section along more than 70% of the circumference of the base section. The receiving portion can comprise a plurality of upper rim sections, particularly a plurality of separate upper rim sections.

In an embodiment, the receiving portion tapers from the upper rim section towards the base section. The recess can widen from the base section towards the upper rim section. The obtuse angle can be between 90° and 180°, particularly between 90° and 170°. With respect to the base section, the border section can have an inclination of more than planar and less than orthogonal.

In an embodiment, the border section comprises a curved portion. The curved portion can be curved from the upper rim section towards the base section. According to an embodiment, the border section is curved from the upper rim section towards the base section. A tangent of the curved portion of the border section and the base section can enclose the obtuse angle. In particular, each tangent of the curved portion of the border section can enclose an obtuse angle with the base section.

The border section and the base section can enclose an obtuse angle. The border section and the base section can enclose the obtuse angle at any position along a contact line between the border section and the base section. In an embodiment, the border section and the base section enclose the obtuse angle at any position along the entire contact line between border section and base section. The tangent of the curved portion of the border section and the base section can enclose the obtuse angle at any position along a contact line between curved portion of the border section and base section, particularly at any position along the entire contact line.

In an embodiment, the border section can extend between the upper rim section and a lower rim section. In an embodiment, the border section and the base section can enclose the obtuse angle at any position along the lower rim section. In an embodiment, the border section and the base section can enclose the obtuse angle at any position along the lower rim section along the circumference of the base section. In an embodiment, the lower rim section can span a virtual base section (virtual base plane). In an embodiment, the border section can enclose an obtuse angle with the virtual base section.

The receiving portion can be configured undercut-less. The undercut-less configuration of the receiving portion can advantageously simplify the cleaning of the receiving portion. The time required for cleaning can advantageously be reduced.

The tapered shape can simplify the insertion of the inserter portion into the receiving portion, e.g. by supporting a sliding in of the inserter portion into the receiving portion. A connection process (assembly of the inserter portion and the receiving portion) can advantageously be supported. The tapered shape can support a sliding off of the inserter portion from the receiving portion. A disconnection process (disassembly of the connected connector system into the subunits inserter portion and receiving portion) can advantageously be supported.

The edge section of the inserter portion can protrude from the bottom section of the inserter portion. The bottom section can extend in a bottom plane (bottom extension plane). The edge section can protrude from the bottom plane. The edge section can be arranged angled to the bottom plane. The inserter portion can taper towards the bottom plane.

In an embodiment, the edge section surrounds a portion of the bottom section along the circumference of the bottom section. In an embodiment, the edge section surrounds the bottom section along more than 30% of the circumference of the bottom section. In an embodiment, the edge section surrounds the bottom section along more than 50% of the circumference of the bottom section. In an embodiment, the edge section surrounds the bottom section along more than 70% of the circumference of the bottom section. In an embodiment, the second connector part can comprise a plurality of edge sections, particularly a plurality of separate edge sections.

In an embodiment, the plurality of separate upper rim sections corresponds to the plurality of separate edge sections.

The edge section and the bottom section can enclose a further obtuse angle. The further obtuse angle enclosed by the edge section and the bottom section can be equal to the obtuse angle enclosed by the border section and the base section.

In an embodiment, the edge section can extend between an upper edge section and a lower edge section. In an embodiment, the lower edge section can span a virtual bottom section (virtual bottom plane). In an embodiment, the edge section and the virtual bottom section can enclose the further obtuse angle. The inserter portion can taper towards the virtual bottom section.

In an embodiment, the inserter portion comprises a top section, the bottom section and the edge section, wherein the edge section connects the top section and the bottom section. The lower edge section can be arranged at the bottom section. The upper edge section can be arranged at the top section.

When the inserter portion is inserted in the receiving portion in the pre-determined orientation, the edge section can face towards the border section. When the inserter portion is inserted in the receiving portion in the pre-determined orientation, the edge section and the border section can abut. The edge section can be complementary to the border section. The edge section and the border section can be complementary for providing mutual surface-wise contact at substantial parts of the respective surfaces. In an embodiment, the edge section and the border section are configured for providing mutual surface-wise contact at the entire surface.

In an embodiment, the receiving portion is complementary to the inserter portion, particularly the shape of the receiving portion is complementary to the shape of the inserter portion. In an embodiment, the shape of the receiving portion is complementary to the shape of the inserter portion at the surface portions contacting each other.

According to an embodiment, a first border section is configured complementary to a first edge section such that the first border section and the first edge section abut, when the inserter portion is arranged in the receiving portion in the pre-determined orientation. The obtuse angle related to the first border section can be equal to the further obtuse angle related to the first edge section. A lateral shifting of the inserter portion within the receiving portion can advantageously be reduced. An advantage can be that the position of the inserter portion within the receiving portion can be stabilised against lateral displacement. An advantage can be that the position of the inserter portion within the receiving portion can be stabilised against rotational displacement.

In the pre-determined orientation, the inserter portion and the receiving portion can establish a form-locked connection. In an embodiment, the inserter portion and the receiving portion are configured to establish a form-locked connection in the pre-determined orientation. In an embodiment, the inserter portion and the receiving portion are configured such that a lateral displacement of the inserter portion with respect to the receiving portion is prevented in the pre-determined orientation. In an embodiment, the inserter portion and the receiving portion are configured such that a rotational displacement of the inserter portion with respect to the receiving portion is prevented in the pre-determined orientation.

In an embodiment, a portion of the inserter portion and a corresponding portion of the receiving portion are configured to establish a form-locked connection, when the inserter portion is arranged in the receiving portion in the pre-determined orientation.

In an embodiment, the connector system is configured such that for connection (assembly, mounting), the inserter portion can be slidingly inserted into the receiving portion. In an embodiment, the connector system is configured such that for disconnection (disassembly, removal), the inserter portion can be lifted out of the receiving portion. In an embodiment, the connector system is configured such that for disconnection, the inserter portion can be levered out of the receiving portion. The connector system can be configured such that the assembly can be performed without screwing the inserter portion into the receiving portion. The connector system can be configured such that the disassembly can be performed without unscrewing the inserter portion from the receiving portion.

An advantage can be that the assembly/disassembly is easy and user-friendly, particularly while still providing a reliable fixing in the pre-determined orientation.

According to an embodiment, the receiving portion is magnetically compatible with the inserter portion for providing a stable attractive magnetic connection, when the inserter portion is arranged in the receiving portion in the pre-determined orientation.

The magnetic element of the receiving portion can comprise a ferromagnetic metal. The magnetic element of the receiving portion can be a permanent magnet. The magnetic element of the inserter portion can comprise a ferromagnetic metal. The magnetic element of the inserter portion can be a permanent magnet. In an embodiment, at least one of the magnetic element of the receiving portion and the magnetic element of the inserter portion is a permanent magnet. In an embodiment, the magnetic element of the receiving portion is a permanent magnet and the magnetic element of the inserter portion is a permanent magnet.

In an embodiment, the magnetic elements are arranged such that the magnetic element of the inserter portion aligns with the magnetic element of the receiving portion, when the inserter portion is received by the receiving portion is the pre-determined orientation. In an embodiment, the polarity of the magnetic element of the inserter portion is opposite to the polarity of the magnetic element of the receiving portion.

The form-fit of the edge section and the border section can provide a torsion resistance of the inserter portion relative to the receiving portion, when the inserter portion is received by the receiving portion in the pre-determined orientation. The magnetic connection between the magnetic element of the inserter portion and the receiving portion can provide a torsion resistance of the inserter portion relative to the receiving portion, when the inserter portion is received by the receiving portion in the pre-determined orientation.

The form-fit and/or the magnetic connection can provide a torsion resistance of the inserter portion relative to the receiving portion, when the inserter portion is received by the receiving portion in the pre-determined orientation. The form-fit and/or the magnetic connection can prevent a rotation of the inserter portion relative to the receiving portion, when the inserter portion is received by the receiving portion in the pre-determined orientation. The form-fit and/or the magnetic connection can prevent a shifting (lateral movement) of the inserter portion relative to the receiving portion, when the inserter portion is received by the receiving portion in the pre-determined orientation. The form-fit and/or the magnetic connection can prevent a displacement of the inserter portion relative to the receiving portion, when the inserter portion is received by the receiving portion in the pre-determined orientation. The form-fit and/or the magnetic connection can provide an accurate spatial alignment of the inserter portion relative to the receiving portion, when the inserter portion is received by the receiving portion in the pre-determined orientation.

In advantage can be that the same relative alignment (of the inserter portion with respect to the receiving portion) can be easily and reliable achieved and locked. In advantage can be that after a replacement of the add-on unit, no new calibration may be necessary. The replacement process can advantageously be simplified and accelerated.

In an embodiment, the first connector part is arrangeable at a housing of the add-on unit. An advantage can be that a cost-efficient upgrading of already available add-on units may be possible.

In an embodiment, the first connector part is integral with the housing of the add-on unit. An advantage can be that the connector system is small and compact.

The housing of the add-on unit can, e.g., house a sensor of the add-on unit. The housing of the add-on unit can support the optical target of the add-on unit.

In an embodiment, the second connector part is integral with a housing of the medical imaging device. The housing of the medical imaging device can house an ultrasonic probe.

In an embodiment, the add-on unit is an add-on tracking device. In an embodiment, the add-on unit is an add-on tracking target.

The add-on tracking target (tracking target) can be an active tracking target. The add-on tracking target (tracking target) can be a passive tracking target. In an embodiment, the add-on tracking target can be an optical target.

An active tracking target can be configured to emit light. An active tracking target can be configured to emit an electromagnetic signal. A passive tracking target can be configured to reflect light. In an embodiment, the the position and/or the rotation of the tracking target is detected by an external measuring device, in particular wherein the external measuring device is configured to determine the tracked position/rotation based on the detected position and/or rotation.

In an embodiment, the add-on unit is a navigation device. The navigation device can be a laser navigation device. The navigation device can comprise a display. The navigation device can comprise a laser projector.

In an embodiment, the add-on unit is an illumination device. The illumination device can comprise a LED light.

In an embodiment, the hand-held medical imaging device is a hand-held ultrasonic device.

In an embodiment, the add-on unit is an add-on tracking device and the hand-held medical imaging device is a hand-held ultrasonic device.

The hand-held medical imaging device can be a tracked hand-held medical imaging device. In an embodiment, the hand-held medical imaging device is a tracked hand-held ultrasonic device.

The hand-held medical imaging device can be a free-hand medical imaging device. The hand-held medical imaging device can be a free-hand movable medical imaging device. In an embodiment, the hand-held medical imaging device is a diagnostic hand-held medical imaging device. The hand-held medical imaging device can be an ultrasonic device (ultrasound). The hand-held medical imaging device can be an opto-acoustic device. The hand-held medical device can be free-hand medical scanner.

The add-on unit, particularly the add-on tracking device, can be configured for providing positional information of the hand-held medical imaging device, when the add-on tracking device is arranged at the medical imaging device. The add-on unit, particularly the add-on tracking device, can be configured for tracking the motion of the hand-held medical imaging device, when the add-on unit is arranged at the hand-held medical imaging device. The motion of the hand-held medical imaging device can comprise a translational movement, a rotational movement and/or a tilting.

The add-on unit can be configured to track an absolute position of the hand-held medical imaging device in a space. The add-on unit can be configured to track a relative position of the hand-held medical imaging device, particularly a positional change of the hand-held medical imaging device. The add-on unit can be configured to track the position, the speed and/or the orientation of the hand-held medical imaging device, particularly of the ultrasonic device. The add-on unit can be configured to track the distance between two positions (e.g. start position and end position) during a scan process.

The add-on unit can comprise a sensor. The add-on unit can comprise a housing configured for housing the sensor. An advantage can be that no additional external sensors may be necessary for tracking. An advantage can be that the use of the medical imaging device can be less restricted to a particular location, for instance, it may easily be used is different consulting rooms.

The add-on unit can comprise a plurality of sensors. A first sensor of the plurality of sensors can be fixed to a second sensor of the plurality of sensors. The first sensor and the second sensor can be arranged in a fixed, pre-defined orientation and/or distance to each other. The first sensor and the second sensor can be fixed in a relative arrangement with respect to another, particularly such that there is no relative spatial movement or rotation between the first sensor and the second sensor. In an embodiment the first sensor and the second sensor are arranged such with respect to each other that a movement and/or rotation of the first sensor implies that the same movement and/or rotation is experienced by the second sensor. In an embodiment, the first sensor is arranged with a pre-defined offset to the second sensor.

The add-on unit can comprise an inertial sensor. The add-on unit can comprise an optical target. The optical target can comprise a QR-code. The optical target can comprise a plurality of spheres. The add-on unit can be configured for optical tracking. The add-on unit can be configured for electromagnetic tracking.

In an embodiment, the add-on unit comprises an inertial measurement unit. In an embodiment, the add-on unit comprises an accelerometer. In an embodiment, the add-on unit comprises a gyroscope. In an embodiment, the add-on unit comprises a magnetometer.

The add-on unit can comprise an optical flow sensor. In an embodiment, the optical flow sensor is configured for generating optical flow data indicative of a two-dimensional movement. The optical flow sensor can be configured for generating optical flow data indicative of a two-dimensional movement over a skin surface of the body portion. The optical flow sensor can be a light detection and ranging (LiDAR) sensor. The optical flow sensor can be a radar sensor. The optical flow sensor can be a camera. The optical flow sensor can be an optical tracking sensor.

In an embodiment, the add-on unit comprises a distance sensor. The distance sensor can be configured for generating distance measurement data. In an embodiment, the distance sensor can be configured for generating distance measurement data indicative of a distance between the distance sensor and the skin surface of the body portion.

In an embodiment, the distance sensor is a non-contact distance sensor. In an embodiment, the distance sensor is an optical time-of-flight sensor. In an embodiment, the distance sensor is a laser distance sensor. The distance sensor can be a laser distance sensor which determines a distance based on the time-of-flight of a reflected laser pulse.

In an embodiment, the add-on unit comprises a distance sensor and an optical flow sensor. In an embodiment, the distance sensor and the optical flow sensor are arranged in a fixed, pre-defined orientation and/or distance to each other.

By relaying information about the distance and speed travelled across the skin using optical flow data, and by adjusting the optical flow data using a distance sensor, the motion of the hand-held medical imaging device can advantageously be determined with improved accuracy.

In an embodiment, the add-on unit can be configured to determine a motion path of the hand-held medical imaging device. For instance, the motion path can be the motion of the hand-held medical imaging device (e.g. the ultrasonic device) across a surface of a body portion during acquisition of an image of the body portion (e.g. an ultrasound image).

The add-on tracking device can be configured to determine the positional information of the hand-held medical imaging device. The add-on tracking device can be configured to provide data to a computing device configured to determine the positional information based on the received data.

In an embodiment, the computing device is configured to generate a 3D tomographic image of a body portion based on data provided by the hand-held medical imaging device and data provided by the add-on unit. In particular, the computing device can be configured to generate a 3D tomographic image of the body portion based on ultrasound image data and the tracked positional information of the ultrasonic device (e.g. the motion path).

In an embodiment, the add-on unit comprises a wireless charging unit for the hand-held medical imaging device.

In an embodiment, the receiving portion extends from a front face of the first connector part towards an opposing foot face of the first connector part, wherein a depth of the receiving portion increases from the front face towards the foot face.

The depth of the receiving portion can increase continuously from the front face towards the foot face. The depth of the receiving portion can increase stepless from the front face towards the foot face.

The depth can be the distance between the base section and the upper rim section, particularly perpendicular to the base section.

The recess can get lower towards the front face. The recess can comprise a deep region and a flat region. In the deep region, the depth can be greater than in the flat region.

An advantage can be that the deep region can provide a good support for the inserter portion, when the inserter portion is inserted in the receiving portion in the pre-determined orientation. An advantage can be that the flat region requires little space such that more space can be available for other entities, e.g. a sensor.

According to an embodiment, the receiving portion comprises a first magnetic element of the receiving portion and a spatially separated second magnetic element of the receiving portion, wherein a polarity of the first magnetic element of the receiving portion is opposite to a polarity of the second magnetic element of the receiving portion.

In an embodiment, the inserter portion comprises a first magnetic element of the inserter portion and a spatially separated second magnetic element of the inserter portion, wherein a polarity of the first magnetic element of the inserter portion is opposite to a polarity of the second magnetic element of the inserter portion.

In an embodiment, the receiving portion comprises a first magnetic element of the receiving portion and a spatially separated second magnetic element of the receiving portion, wherein a polarity of the first magnetic element of the receiving portion is opposite to a polarity of the second magnetic element of the receiving portion, and the inserter portion comprises a first magnetic element of the inserter portion and a spatially separated second magnetic element of the inserter portion, wherein a polarity of the first magnetic element of the inserter portion is opposite to a polarity of the second magnetic element of the inserter portion.

In an embodiment, the inserter portion and the receiving portion are configured such that the first magnetic element of the inserter portion aligns with the first magnetic element of the receiving portion, when the inserter portion is received by the receiving portion in the pre-determined orientation, wherein the polarity of the first magnetic element of the inserter portion is opposite to the polarity of the first magnetic element of the receiving portion.

In an embodiment, the inserter portion and the receiving portion are configured such that the second magnetic element of the inserter portion aligns with the second magnetic element of the receiving portion, when the inserter portion is received by the receiving portion in the pre-determined orientation, wherein the polarity of the second magnetic element of the inserter portion is opposite to the polarity of the second magnetic element of the receiving portion.

According to an embodiment, the inserter portion and the receiving portion are configured such that the first magnetic element of the inserter portion aligns with the first magnetic element of the receiving portion, when the inserter portion is received by the receiving portion in the pre-determined orientation, wherein the polarity of the first magnetic element of the inserter portion is opposite to the polarity of the first magnetic element of the receiving portion, and the inserter portion and the receiving portion are configured such that the second magnetic element of the inserter portion aligns with the second magnetic element of the receiving portion, when the inserter portion is received by the receiving portion in the pre-determined orientation, wherein the polarity of the second magnetic element of the inserter portion is opposite to the polarity of the second magnetic element of the receiving portion.

The first magnetic element of the receiving portion can be spaced apart from the second magnetic element of the receiving portion. In an embodiment, the first magnetic element of the receiving portion and/or the second magnetic element of the receiving portion require only little space for installation. The space between the first magnetic element of the receiving portion and the second magnetic element of the receiving portion can be available for a further component of the add-on unit (such as a component of a sensor), particularly when the first connector part is integral with the housing of the add-on unit. An advantage can be that the first connector part can be small in size. Advantageously, the first connector part may be convenient to handle. An advantage can be a user-friendly handling of the connector system in the assembled state.

The first magnetic element of the inserter portion can be spaced apart from the second magnetic element of the inserter portion. In an embodiment, the first magnetic element of the inserter portion and/or the second magnetic element of the inserter portion require only little space for installation. Advantageously, the second connector part may be convenient to handle. An advantage can be a user-friendly handling of the connector system in the assembled state.

The fixation (locking) of the inserter portion in the receiving portion in the pre-determined orientation can be enhanced by the plurality of magnetic elements of the receiving portion and the inserter portion. A reasonably strong fixation can be achieved by means of a plurality of weak magnetic elements. An advantage can be that an easily releasable strong fixation can be provided. An advantage can be safe handling (in the assembled state) and, as necessary, an easy replacement of the add-on unit.

During the insertion of the inserter portion into the receiving portion, the different polarities of the first and second magnetic element can advantageously prevent the inserter portion from being locked in an orientation relative to the receiving portion other than the pre-determined orientation. The magnetic elements of the same polarity can repel each other such that a fixation of the inserter portion in the receiving portion can be prevented.

This can advantageously provide an easy and reliable guide towards the pre-determined orientation. Advantageously, the connecting process can be improved.

In an embodiment, the receiving portion comprises a third magnetic element arranged spatially separated to the first magnetic element and the second magnetic element. In an embodiment, the first magnetic element, the second magnetic element and the third magnetic element are arranged such that they form the corners of a triangle (particularly parallel to the base plane).

In an embodiment, the inserter portion comprises a third magnetic element of the inserter portion arranged spatially separated to the first magnetic element of the inserter portion and the second magnetic element of the inserter portion. In an embodiment, the first magnetic element of the inserter portion, the second magnetic element of the inserter portion and the third magnetic element of the inserter portion are arranged such that they form the corners of a triangle (particularly parallel to the bottom plane).

In an embodiment, the third magnetic element of the receiving portion is arranged in the support section of the receiving portion. In an embodiment, the third magnetic element of the inserter portion is arranged in the support section of the inserter portion.

In an embodiment, the third magnetic element of the receiving portion is arranged in the central section of the receiving portion. In an embodiment, the third magnetic element of the inserter portion is arranged in the central section of the inserter portion.

In an embodiment, the third magnetic element of the receiving portion aligns with the third magnetic element of the inserter portion, when the inserter portion is received in the receiving portion is the pre-determined orientation. In an embodiment, the polarity of the third magnetic element of the receiving portion is opposite to the polarity of the third magnetic element of the inserter portion.

This can advantageously provide an easy and reliable guide towards the pre-determined orientation. Advantageously, the connecting process can be improved.

In an embodiment, the receiving portion extends from a front face of the first connector part towards an opposing foot face of the first connector part along a longitudinal direction of the receiving portion, wherein the shape of the receiving portion is reflection symmetric with respect to a centre axis of the receiving portion extending along the longitudinal direction of the receiving portion.

In an embodiment, the inserter portion extents from a bridging section of the second connector part towards an opposing tip along a longitudinal direction of the inserter portion, wherein the shape of the inserter portion is reflection symmetric with respect to a centre axis of the inserter portion extending along the longitudinal direction of the inserter portion.

According to an embodiment, the receiving portion extends from a front face of the first connector part towards an opposing foot face of the first connector part along a longitudinal direction of the receiving portion, wherein the shape of the receiving portion is reflection symmetric with respect to a centre axis of the receiving portion extending along the longitudinal direction of the receiving portion, and the inserter portion extents from a bridging section of the second connector part towards an opposing tip along a longitudinal direction of the inserter portion, wherein the shape of the inserter portion is reflection symmetric with respect to a centre axis of the inserter portion extending along the longitudinal direction of the inserter portion.

The shape of the receiving portion can be mirror symmetric with respect to the centre axis of the receiving portion. The shape of the inserter portion can be mirror symmetric with respect to the centre axis of the inserter portion.

In the connected state, in the pre-determined orientation, the centre axis of the inserter portion and the centre axis of the receiving portion may align with each other.

In an embodiment, the shape of the receiving portion is not rotationally symmetrical. In an embodiment, the shape of the inserter portion is not rotationally symmetrical.

An advantage can be to provide the unique pre-determined orientation. An advantage can be that an orientation deviating from the pre-determined orientation of the receiving portion with respect to the inserter portion is prevented. The unique positioning can be made possible. A new calibration (e.g. after replacement of the add-on unit) can become advantageously unnecessary. A possible exchange of the add-on unit can be simplified.

In an embodiment, the receiving portion comprises a support section of the receiving portion, a central section of the receiving portion, a first lateral section of the receiving portion and a second lateral section of the receiving portion, wherein the first lateral section of the receiving portion and the second lateral section of the receiving portion protrude laterally beyond the central section of the receiving portion.

In an embodiment, the border section and the base section enclose a larger obtuse angle in the first lateral section of the receiving portion than in the support section of the receiving portion. In an embodiment, the border section and the base section enclose a larger obtuse angle in the second lateral section of the receiving portion than in the support section of the receiving portion. In an embodiment, the border section and the base section enclose a larger obtuse angle in the first lateral section of the receiving portion and in the second lateral section of the receiving portion than in the support section of the receiving portion.

In an embodiment, the inserter portion comprises a support section of the inserter portion, a central section of the inserter portion, a first lateral section of the inserter portion and a second lateral section of the inserter portion, wherein the first lateral section of the inserter portion and the second lateral section of the inserter portion protrude laterally beyond the central section of the inserter portion.

In an embodiment, the edge section and the bottom section enclose a larger obtuse angle in the first lateral section of the inserter portion than in the support section of the inserter portion. In an embodiment, the edge section and the bottom section enclose a larger obtuse angle in the second lateral section of the inserter portion than in the support section of the inserter portion. In an embodiment, the edge section and the bottom section enclose a larger obtuse angle in the first lateral section of the inserter portion and in the second lateral section of the inserter portion than in the support section of the inserter portion.

In an embodiment, the border section and the base section enclose a larger obtuse angle in the first lateral section of the receiving portion and in the second lateral section of the receiving portion than in the support section of the receiving portion, and the edge section and the bottom section enclose a larger obtuse angle in the first lateral section of the inserter portion and in the second lateral section of the inserter portion than in the support section of the inserter portion.

In an embodiment, the border section and the base section enclose a larger obtuse angle in the first lateral section of the receiving portion than in the support section of the receiving portion, and the edge section and the bottom section enclose a larger obtuse angle in the first lateral section of the inserter portion than in the support section of the inserter portion.

In an embodiment, the border section and the base section enclose a larger obtuse angle in the second lateral section of the receiving portion than in the support section of the receiving portion, and the edge section and the bottom section enclose a larger obtuse angle in the second lateral section of the inserter portion than in the support section of the inserter portion.

Along the circumference of the base section, the obtuse angle can change, particularly can change continuously. Along the extension of the upper rim section, the obtuse angle can change, particularly can change continuously.

The first lateral section of the receiving portion can face away from the second lateral section of the receiving portion. The first lateral section of the receiving portion and the second lateral section of the receiving portion can extend in different directions, particularly relative to the centre axis of the receiving portion. Perpendicular to the surface, the upper rim section of the first lateral section can be more distant to the centre axis of the receiving portion than the upper rim section of support section. Perpendicular to the surface, the upper rim section of the second lateral section can be more distant to the centre axis of the receiving portion than the upper rim section of support section.

The first lateral section of the inserter portion can face away from the second lateral section of the inserter portion. The first lateral section of the inserter portion and the second lateral section of the inserter portion can extend in different directions, particularly relative to the centre axis of the inserter portion. Perpendicular to the bottom section, the edge section of the first lateral section can be more distant to the centre axis of the inserter portion than the edge section of support section. Perpendicular to the bottom section, the edge section of the second lateral section can be more distant to the centre axis of the inserter portion than the edge section of support section.

The central section of the receiving portion can be adjacent to the support section of the receiving portion. Along the longitudinal direction of the receiving portion, the central section of the receiving portion can be rear to the support section of the receiving portion. The central section of the receiving portion can be closer to the foot face of the first connector part than the support section of the receiving portion. The first lateral section of the receiving portion and the second lateral section of the receiving portion can be arranged closer to the foot face of the receiving portion than the support section of the receiving portion.

The central section of the inserter portion can be adjacent to the support section of the inserter portion. Along the longitudinal direction of the inserter portion, the central section of the inserter portion can be rear to the support section of the inserter portion. The central section of the inserter portion can be closer to the bridging portion of the second connector part than the support section of the inserter portion. The first lateral section of the inserter portion and the second lateral section of the inserter portion can be arranged closer to the tip of the inserter portion than the support section of the inserter portion.

In an embodiment, the border section of the support section of the receiving portion is steeper than the border section in the first lateral section and/or than the border section in the second lateral section of the receiving portion. In an embodiment, the edge section of the support section of the inserter portion is steeper than the border section in the first lateral section and/or than the border section in the second lateral section of the inserter portion.

An advantage of a larger obtuse angle can be an easier cleaning, particularly an easier cleaning of the receiving portion. An advantage of the larger obtuse angle can be an easier sliding in of the inserter portion into the receiving portion. An advantage of the larger obtuse angle can be an easier sliding out of the inserter portion from the receiving portion. Advantageously, the assembly and/or disassembly can be facilitated. Advantageously, the cleaning of the connector system can be facilitated and downtimes due to cleaning can be reduced.

An advantage of the smaller obtuse angle can be a better support of the inserter portion by the receiving portion in the predetermined orientation (in the assembled state). A lateral movement of the inserter portion relative to the receiving portion can advantageously be prevented. A rotational movement of the inserter portion relative to the receiving portion can advantageously be prevented. Advantageously, keeping the pre-determined orientation can be supported by the smaller obtuse angle in the support section, particularly by the steeper border section and the steeper edge section in the respective support sections.

The combination of portions with a larger obtuse angle and portions with a smaller obtuse angle can advantageously provide a good support in the assembled state as well as an easy assembly and/or disassembly and/or cleaning.

In an embodiment, the depth of the first lateral section of the receiving portion is greater than the depth of the support section of the receiving portion. In an embodiment, the depth of the second lateral section of the receiving portion is greater than the depth of the support section of the receiving portion.

The border section and the base section can enclose an obtuse angle. The edge section and the bottom section can enclose a further obtuse angle. The further obtuse angle enclosed by the edge section and the bottom section can be equal to the obtuse angle enclosed by the border section and the base section.

In an embodiment, the further obtuse angle in the support section of the inserter portion equals the respective obtuse angle in the support section of the receiving portion. In an embodiment, the further obtuse angle in the first lateral section of the inserter portion equals the respective obtuse angle in the first lateral section of the receiving portion. In an embodiment, the further obtuse angle in the second lateral section of the inserter portion equals the respective obtuse angle in the second lateral section of the receiving portion.

In an embodiment, along the entire circumference of the receiving portion and the entire circumference of the inserter portion, the further obtuse angle equals the obtuse angle. In an embodiment, in the assembled state (in the pre-determined orientation), the border section and the edge section can abut, particularly along the entire circumference of the receiving portion and the entire circumference of the inserter portion.

An advantage can be an enhanced form-locked connection (when the inserter portion is arranged in the receiving portion in the pre- determined orientation).

In an embodiment, the further obtuse angle of a section of the inserter portion equals the obtuse angle of a corresponding section of the receiving portion. In an embodiment, the further obtuse angle of a section of the inserter portion equals the obtuse angle of a corresponding section of the receiving portion, and the further obtuse angle of a further section of the inserter portion differs from the obtuse angle of a corresponding further section of the receiving portion.

In an embodiment, the further obtuse angle in the support section of the inserter portion equals the respective obtuse angle in the support section of the receiving portion, and the further obtuse angle in the first lateral section of the inserter portion is smaller than the respective obtuse angle in the first lateral section of the receiving portion. In an embodiment, the further obtuse angle in the support section of the inserter portion equals the respective obtuse angle in the support section of the receiving portion, and the further obtuse angle in the second lateral section of the inserter portion is smaller than the respective obtuse angle in the second lateral section of the receiving portion.

In an embodiment, in the assembled state (in the pre-determined orientation), the outline of the bottom section of the inserter portion abuts the lower rim section (or contact line) of the receiving portion and the border section of the support section abuts the respective edge section of the support section. In an embodiment, in the assembled state (in the pre-determined orientation), the outline of the bottom section of the inserter portion abuts the lower rim section (or contact line) of the receiving portion, the border section of the support section abuts the respective edge section of the support section, and the border section of the first lateral section is distant to the respective edge section of the first lateral section. In an embodiment, in the assembled state (in the pre-determined orientation), the outline of the bottom section of the inserter portion abuts the lower rim section (or contact line) of the receiving portion, the border section of the support section abuts the respective edge section of the support section, and the border section of the second lateral section is distant to the respective edge section of the second lateral section.

An advantage can be a simplified insertion/release while maintaining an appropriate connection when the inserter portion is arranged in the receiving portion in the pre-defined orientation.

According to an embodiment, the receiving portion comprises a support section, a central section, a first lateral section and a second lateral section, wherein the first lateral section and the second lateral section protrude laterally beyond the central section, wherein the receiving portion has a first concave upper rim section between the first lateral section of the receiving portion and the second lateral section of the receiving portion.

In an embodiment, the receiving portion comprises a support section, a central section, a first lateral section and a second lateral section, wherein the first lateral section and the second lateral section protrude laterally beyond the central section, wherein the receiving portion has a second concave upper rim section between the first lateral section of the receiving portion and the support section of the receiving portion and a third concave upper rim section between the second lateral section of the receiving portion and the support section of the receiving portion.

In an embodiment, the receiving portion comprises a support section, a central section, a first lateral section and a second lateral section, wherein the first lateral section and the second lateral section protrude laterally beyond the central section, wherein the first lateral section of the receiving portion has a convex upper rim section.

In an embodiment, the receiving portion comprises a support section, a central section, a first lateral section and a second lateral section, wherein the first lateral section and the second lateral section protrude laterally beyond the central section, wherein the second lateral section of the receiving portion has a convex upper rim section.

According to an embodiment, the receiving portion comprises a support section, a central section, a first lateral section and a second lateral section, wherein the first lateral section and the second lateral section protrude laterally beyond the central section, wherein the receiving portion has a first concave upper rim section between the first lateral section of the receiving portion and the second lateral section of the receiving portion, and wherein the receiving portion has a second concave upper rim section between the first lateral section of the receiving portion and the support section of the receiving portion and a third concave upper rim section between the second lateral section of the receiving portion and the support section of the receiving portion.

According to an embodiment, the receiving portion comprises a support section, a central section, a first lateral section and a second lateral section, wherein the first lateral section and the second lateral section protrude laterally beyond the central section, wherein the receiving portion has a first concave upper rim section between the first lateral section of the receiving portion and the second lateral section of the receiving portion, wherein the receiving portion has a second concave upper rim section between the first lateral section of the receiving portion and the support section of the receiving portion and a third concave upper rim section between the second lateral section of the receiving portion and the support section of the receiving portion, wherein the first lateral section of the receiving portion has a convex upper rim section, and wherein the second lateral section of the receiving portion has a convex upper rim section.

The first lateral section of the receiving portion can be connected with the second lateral section of the receiving portion by the first concave upper rim section. The first concave upper rim section can be curved towards the support section of the receiving portion. The first concave upper rim section can be curved towards the front face of the receiving portion.

In an embodiment, a wireless charging coil of the add-on unit is arranged to be at least partially surrounded by and received within the first concave upper rim section.

The second concave upper rim section of the receiving portion can connect the first lateral section of the receiving portion and the support section of the receiving portion. The third concave upper rim section can connect the second lateral section of the receiving portion and the support section of the receiving portion. The second concave upper rim section can be curved towards the centre axis. The third concave upper rim section can be curved towards the centre axis. The second concave upper rim section and the third concave upper rim section can be curved towards each other.

The convex upper rim section can be curved away from the central section. The convex upper rim section can be curved away from the centre axis.

In an embodiment, the first magnetic element is arranged in the first lateral section of the receiving portion. The first magnetic element can be at least partially surrounded by the convex upper rim section of the first lateral section. In an embodiment, the second magnetic element is arranged in the second lateral section of the receiving portion. The second magnetic element can be at least partially surrounded by the convex upper rim section of the second lateral section.

The curved trace of the upper rim section can be the contour of the receiving portion, particularly the contour of the receiving portion in the surface plane.

In an embodiment, the first lateral section and the second lateral section can protrude laterally beyond the support section. In an embodiment, close to the foot face, the receiving portion is wider than close to the front face.

In an embodiment, the inserter portion comprises a support section, a central section, a first lateral section and a second lateral section, wherein the first lateral section and the second lateral section protrude laterally beyond the central section, wherein the inserter portion has a first concave contour section between the first lateral section of the inserter portion and the second lateral section of the inserter portion.

In an embodiment, the inserter portion comprises a support section, a central section, a first lateral section and a second lateral section, wherein the first lateral section and the second lateral section protrude laterally beyond the central section, wherein the inserter portion has a second concave contour section between the first lateral section of the inserter portion and the support section of the inserter portion and a third concave contour section between the second lateral section of the inserter portion and the support section of the inserter portion.

In an embodiment, the inserter portion comprises a support section, a central section, a first lateral section and a second lateral section, wherein the first lateral section and the second lateral section protrude laterally beyond the central section, wherein the first lateral section of the inserter portion has a convex contour section.

In an embodiment, the inserter portion comprises a support section, a central section, a first lateral section and a second lateral section, wherein the first lateral section and the second lateral section protrude laterally beyond the central section, wherein the second lateral section of the inserter portion has a convex contour section.

In an embodiment, the inserter portion comprises a support section, a central section, a first lateral section and a second lateral section, wherein the first lateral section and the second lateral section protrude laterally beyond the central section, wherein the inserter portion has a first concave contour section between the first lateral section of the inserter portion and the second lateral section of the inserter portion, and wherein the inserter portion has a second concave contour section between the first lateral section of the inserter portion and the support section of the inserter portion and a third concave contour section between the second lateral section of the inserter portion and the support section of the inserter portion.

In an embodiment, the inserter portion comprises a support section, a central section, a first lateral section and a second lateral section, wherein the first lateral section and the second lateral section protrude laterally beyond the central section, wherein the inserter portion has a first concave contour section between the first lateral section of the inserter portion and the second lateral section of the inserter portion, wherein the inserter portion has a second concave contour section between the first lateral section of the inserter portion and the support section of the inserter portion and a third concave contour section between the second lateral section of the inserter portion and the support section of the inserter portion, wherein the first lateral section of the inserter portion has a convex contour section, and wherein the second lateral section of the inserter portion has a convex contour section.

In an embodiment, the first lateral section and the second lateral section can protrude laterally beyond the support section.

The first lateral section of the inserter portion can be connected with the second lateral section of the inserter portion by the first concave contour section. The first concave contour section can be curved towards the support section of the inserter portion. The first concave contour section can be curved towards the bridging section.

The second concave contour section of the inserter portion can connect the first lateral section of the inserter portion and the support section of the inserter portion. The third concave contour section can connect the second lateral section of the inserter portion and the support section of the inserter portion. The second concave contour section can be curved towards the centre axis. The third concave contour section can be curved towards the centre axis. The second concave contour section and the third concave contour section can be curved towards each other.

The convex contour section can be curved away from the central section. The convex contour section can be curved away from the centre axis.

In an embodiment, the first magnetic element is arranged in the first lateral section of the inserter portion. The first magnetic element can be at least partially surrounded by the convex contour section of the first lateral section. In an embodiment, the second magnetic element is arranged in the second lateral section of the inserter portion. The second magnetic element can be at least partially surrounded by the convex contour section of the second lateral section.

Particularly, the respective sections of the receiving portion and the inserter portion correspond to each other. For instance, a length and a curvature of the first concave upper rim section (of the receiving portion) can correspond to a length and curvature of the first concave contour section (of the inserter portion).

In an embodiment, the shape of the receiving portion and the inserter portion is configured such that a large leverage effect can be provided, particularly while keeping the connector system small. A large leverage can be advantageous for the disassembly of the (assembled) connector system.

In an embodiment, the magnetic elements are arranged such that they can be released one after the other.

In an embodiment, the receiving portion forms a slot in a front face of the first connector part. In an embodiment, the support section of the receiving portion forms the slot in the front face of the first connector part, particularly in a housing of the add-on unit.

In an embodiment, the support section of the inserter portion is configured to be at least partially insertable in the slot of the first connector part. In an embodiment, the slot is configured to at least partially receive the support section of the inserter portion.

The insertion of the support section in the slot can contribute to the alignment of the inserter portion in the receiving portion and the locking in the pre-determined orientation. Advantageously, it can contribute to the stabilisation of the position of the inserter portion in the receiving portion in the pre-determined orientation.

According to an embodiment, the inserter portion comprises a top section, wherein the edge section connects the top section and the bottom section, particularly wherein the top section faces away from the bottom section.

In an embodiment, the top section extends in a top extension plane and the bottom section extends in a bottom extension plane, wherein the top extension plane extends parallel to the bottom extension plane.

The top section can extend parallel to the bottom section.

In an embodiment, the second connector part comprises a bridging section connecting a device connector portion and the inserter portion, wherein the device connector portion is configured for repeatedly releasably connecting the second connector part and the hand-held medical imaging device.

The device connector portion can provide an easy mounting of the second connector part to the hand-held medical imaging device, particularly to the ultrasonic device.

In an embodiment, the second connector part comprises a bridging section connecting a device connector portion and the inserter portion, wherein the bottom section faces away from the device connector portion.

In an embodiment, the second connector part comprises a bridging section connecting a device connector portion and the inserter portion, wherein the bottom section and the device connector portion enclose an obtuse angle.

In an embodiment, the second connector part can be configured such that, the add-on unit is spaced apart from the hand-held medical imaging device, when connected via the connector system, particularly spaced apart with a predefined distance. In particular, the add-on unit can be arranged such with respect to the hand-held medical imaging device that the add-on unit is out of the field of view of the hand-held medical imaging device. In particular, the add-on unit can be arranged such with respect to the hand-held medical imaging device that the hand-held medical imaging device is out of the field of view of the add-on unit. Advantageously, a mutual interference can be prevented, particularly a mutual interference of the medical imaging device and the add-on unit.

In an embodiment, the second connector part comprises a bridging section connecting a device connector portion and the inserter portion, wherein the second connector part comprises a spacer, wherein the spacer is arranged and configured such that the spacer point away from the bottom section, particularly wherein the spacer points towards the device connector portion.

In an embodiment, the spacer is arranged and configured such that the spacer abuts a housing of the hand-held medical imaging device, when the second connector part is arranged at the hand-held medical imaging device.

In an embodiment, the spacer is arranged at the top section.

The spacer can stabilise the distance between the add-on unit and the hand-held medical imaging device, when connected via the connector system. The spacer can be arranged and configured to reduce vibrations. The tracking can advantageously be improved.

According to an embodiment, the second connector part comprises a bridging section connecting a device connector portion and the inserter portion, wherein the device connector portion is configured for repeatedly releasably connecting the second connector part and the hand-held medical imaging device, and wherein the bottom section faces away from the device connector portion, and wherein the bottom section and the device connector portion enclose an obtuse angle, and wherein the second connector part comprises a spacer, wherein the spacer is arranged and configured such that the spacer point away from the bottom section, particularly wherein the spacer points towards the device connector portion, particularly wherein the spacer is arranged and configured such that the spacer abuts a housing of the hand-held medical imaging device, when the second connector part is arranged at the hand-held medical imaging device, particularly wherein the spacer is arranged at the top section.

A further aspect of the invention is related to a method for connecting an add-on unit, particularly an add-on tracking device, and a hand-held medical imaging device, using a connector system according to the invention, wherein the connector system is connected with the hand-held medical imaging device, particularly via the device connector portion.

The method for connecting comprises:
- an insertion step, wherein in the insertion step the inserter portion of the medical-imaging-device-side second connector part is inserted in the receiving portion of the add-on unit-side first connector part in the pre-determined orientation, and
- a fixation step, wherein in the fixation step, the magnetic connection between the inserter portion and the receiving portion in the pre-determined orientation is provided by the magnetic elements of the inserter portion and the receiving portion.

The insertion step can comprise a sliding in of the inserter portion into the receiving portion.

A further aspect is related to a method for disconnecting an add-on unit, particularly an add-on tracking device, and a hand-held medical imaging device, from the pre-determined orientation in a connector system according to the invention, wherein the connector system is connected with the hand-held medical imaging device, particularly via the device connector portion.

The method for disconnecting comprises:
- releasing step, wherein in the releasing step, the magnetic connection between the inserter portion and the receiving portion is released by levering off the inserter portion in the receiving portion, and
- a removal step, wherein in the removal step, the inserter portion is removed from the receiving portion.

A further aspect of the invention is related to an add-on-unit-side first connector part for the connector system according to the invention for connecting the add-on unit and the hand-held medical imaging device.

The add-on-unit-side first connector part comprises the recessed receiving portion, wherein the receiving portion is configured for receiving the protruding inserter portion of the medical-imaging-device-side second connector part in the unique pre-determined orientation. The receiving portion comprises the upper rim section, the base section and the border section connecting the upper rim section and the base section, wherein the border section and the base section enclose the obtuse angle.

The border section is arranged and configured such that the border section is form-fit to the edge section of the inserter portion such that the inserter portion is receivable by the receiving portion in the unique pre-determined orientation with respect to each other.

The receiving portion comprises the magnetic element configured to provide the magnetic connection with the inserter portion to fixate the inserter portion in the receiving portion in the pre-determined orientation.

A further aspect of the invention is related to a medical-imaging-device-side second connector part for the connector system according to the invention for connecting the add-on unit and the hand-held medical imaging device.

The medical-imaging-device-side second connector part comprises the protruding inserter portion, wherein the inserter portion is configured for insertion in the recessed receiving portion of the add-on-unit-side first connector part in the unique pre-determined orientation.

The inserter portion comprises the bottom section and the edge section connected to the bottom section, wherein the edge section is arranged and configured such that the edge section is form-fit to the border section of the receiving portion such that the inserter portion is receivable by the receiving portion in the unique pre-determined orientation with respect to each other.

The inserter portion comprises the magnetic element configured to provide the magnetic connection with the receiving portion to fixate the inserter portion in the receiving portion in the pre-determined orientation.

A further aspect of the invention is related to a medical system comprising a hand-held medical imaging device, an add-on unit, and a connector system for connecting the add-on unit and the hand-held medical imaging device, particularly the connector system according to the invention.

The connector system comprises an add-on-unit-side first connector part comprising a recessed receiving portion, and a medical-imaging-device-side second connector part comprising a protruding inserter portion.

The receiving portion comprises an upper rim section, a base section and a border section connecting the upper rim section and the base section, wherein the border section and the base section enclose an obtuse angle. The inserter portion comprises a bottom section and an edge section connected to the bottom section. The edge section is form-fit to the border section such that the inserter portion is receivable by the receiving portion in a unique pre-determined orientation with respect to each other. The receiving portion and the inserter portion each comprises a magnetic element configured to provide a magnetic connection between each other to fixate the inserter portion in the receiving portion in the pre-determined orientation.

In an embodiment, the hand-held medical imaging device is an ultrasonic device.

In an embodiment, the add-on unit is an add-on tracking device. In an embodiment, the add-on unit comprises a sensor. In an embodiment, the add-on unit comprises a plurality of sensors.

According to an embodiment, the hand-held medical imaging device is an ultrasonic device and the add-on unit is an add-on tracking device. According to an embodiment, the hand-held medical imaging device is an ultrasonic device and the add-on unit comprises a sensor.

Further features and embodiments of the present invention are described in the following with reference to the Figures, wherein:
- Fig. 1: shows a schematic side view of an embodiment of a medical system comprising an add-on tracking device, a medical imaging device and a connector system,
- Fig. 2: shows a schematic perspective view of an embodiment of an assembled connector system,
- Fig. 3: shows a schematic bottom view of an embodiment of an add-on tracking device comprising a first connector part,
- Fig. 4A: shows a schematic detailed view of an embodiment of a receiving portion comprising a straight border section,
- Fig. 4B: shows a schematic detailed view of an embodiment of a receiving portion comprising a curved border section,
- Fig. 5: shows the section along A-A from Fig. 3,
- Fig. 6: shows the section along B-B from Fig. 3,
- Fig. 7: shows the section along C-C from Fig. 3,
- Fig. 8: shows a schematic bottom view of an embodiment of a second connector part,
- Fig. 9: shows a schematic perspective view of an embodiment of a second connector part, and
- Fig. 10: shows a schematic side view of an embodiment of a second connector part.

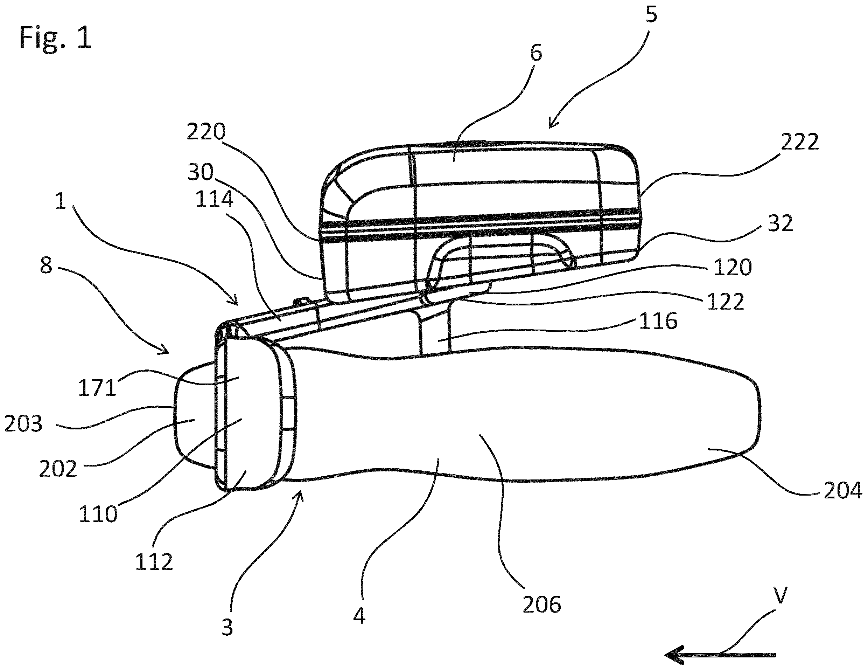

Fig. 1 shows an embodiment of a medical system 8 comprising an add-on unit 5 (particularly an add-on tracking device 5), a medical imaging device 3 and a connector system 1. In the example illustrated in Fig. 1, the medical imaging device 3 is an ultrasonic device (ultrasound). The medical imaging device 3 comprises a housing 4 of the medical imaging device 3. The shown medical imaging device 3 comprises a front section 202, a central section 206 and a rear section 204.

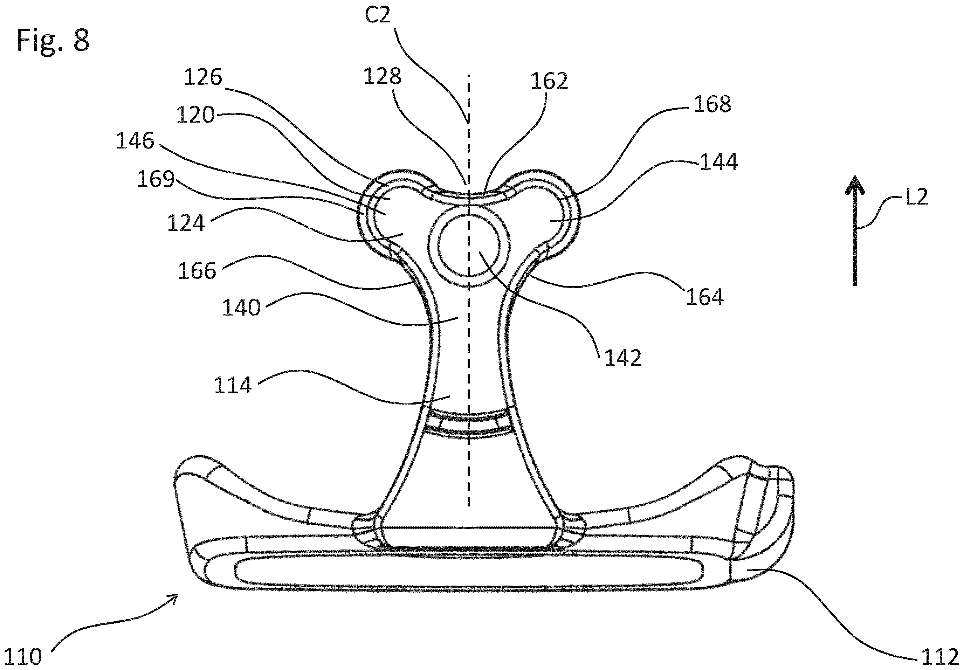

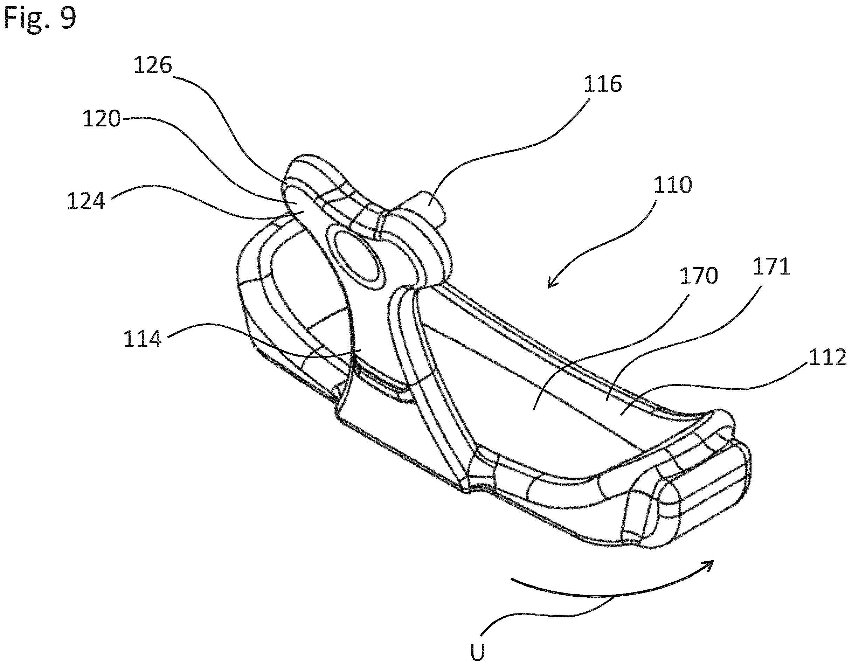

In the illustrated embodiment (Fig. 1), a second connector part 110 is arranged at the medical imaging device 3. In particular, a device connector portion 112 of the second connector part 110 is arranged at the front portion 202 of the medical imaging device 3. In the illustrated embodiment, the second connector part 110 is configured and arranged such at medical imaging device 3 that a bridging portion 114 of the second connector part 110 extends in the direction of the rear section 204 of the medical imaging device 3. In the assembled (connected) state illustrated in Fig. 1, a top section 122 of the inserter portion 120 can face towards the medical imaging device 3. The second connector part 110 can comprise a spacer 116. The spacer 116 can be arranged at the top section 122 of the inserter portion 120 (also see Figs 8, 9, 10). In the assembled (connected) state illustrated in Fig. 1, the spacer 116 can abut the housing 4 of the medical imaging device 3.

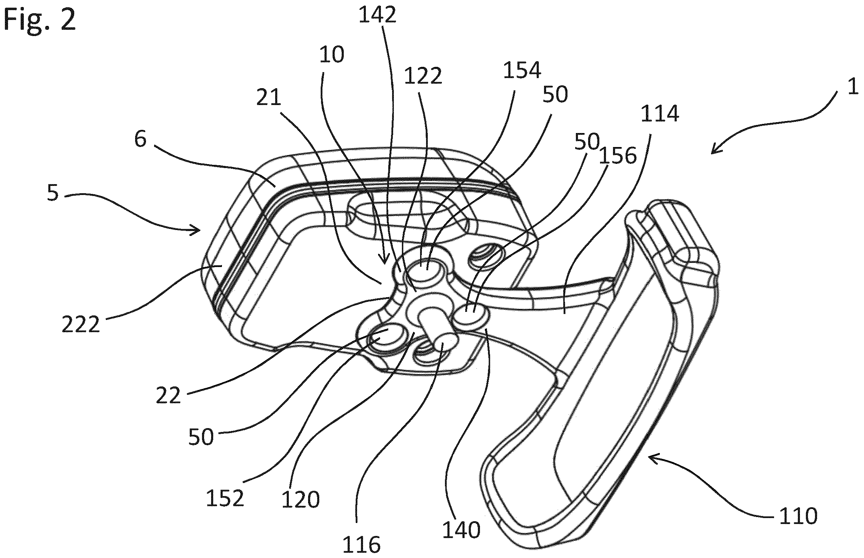

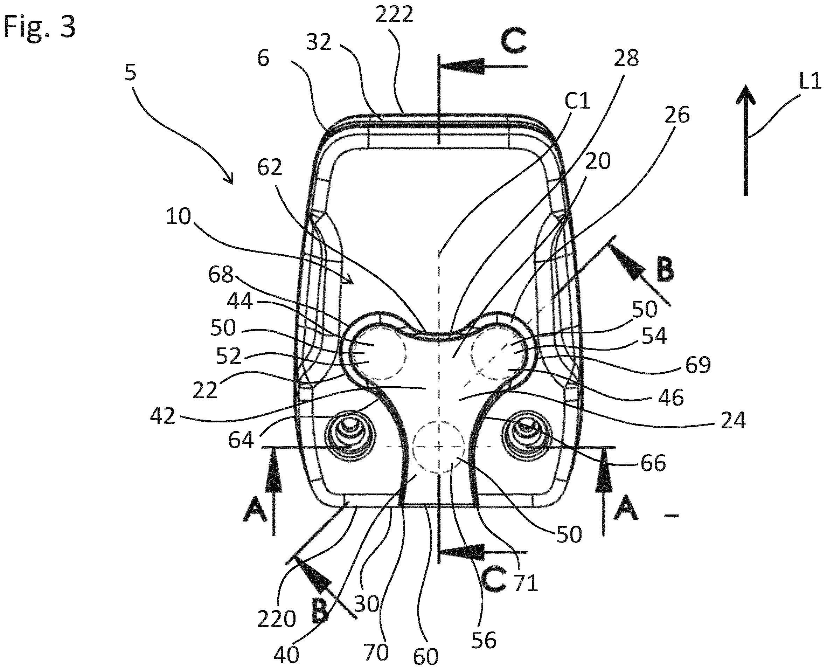

The add-on tracking device 5 can be connected with the second connector part 110, particularly with the inserter portion 120 (also see Fig. 2). The add-on tracking device 5 can comprise a housing 6. The second connector part 110 can be integral with the housing 6 of the add-on tracking device 5 (also see Figs 2 and 3). The add-on tracking device 5 can comprise a front end 220 and an opposing rear end 222. In the presented embodiment, in the connected state, the front end 220 of the add-on tracking device 5 points towards the same view direction V as the front section 202 of the medical imaging device 3.

The bridging portion 114 can extend angled to the view direction V. In particular, the bridging portion 114 can extend such that, when connected, the add-on tracking device 5 is spaced apart from the medical imaging device 3. In particular, the add-on tracking device 5 is spaced apart from the medical imaging device 3 in a pre-defined distance (in particular perpendicular to the view direction V), when connected.

Fig. 2 shows an embodiment of a connected state of a connector system 1. The inserter portion 120 can be inserted in the receiving portion. The inserter portion 120 can contact an upper rim section 22 of the receiving portion. A surface portion of the inserter portion 120 can contact the upper rim section 22 of the receiving portion. An edge section 126 of the inserter portion 120 can contact the upper rim section 22 of the receiving portion.

In the illustrated embodiment, the top section 122 of the inserter portion 120 faces away from the add-on tracking device 5, particularly from the receiving portion, when the inserter portion 120 is received by the receiving portion in the pre-determined orientation.

In Fig. 3, an embodiment of a first connector part 10 comprising a receiving portion 20 is shown. In the illustrated example, the receiving portion 20 is integral with the housing 6 of the add-on tracking device 5. In particular, the receiving portion 20 can be configured as a recess in the housing 6 of the add-on tracking device 5. The receiving portion 20 can arranged at the bottom of the add-on tracking device 5.

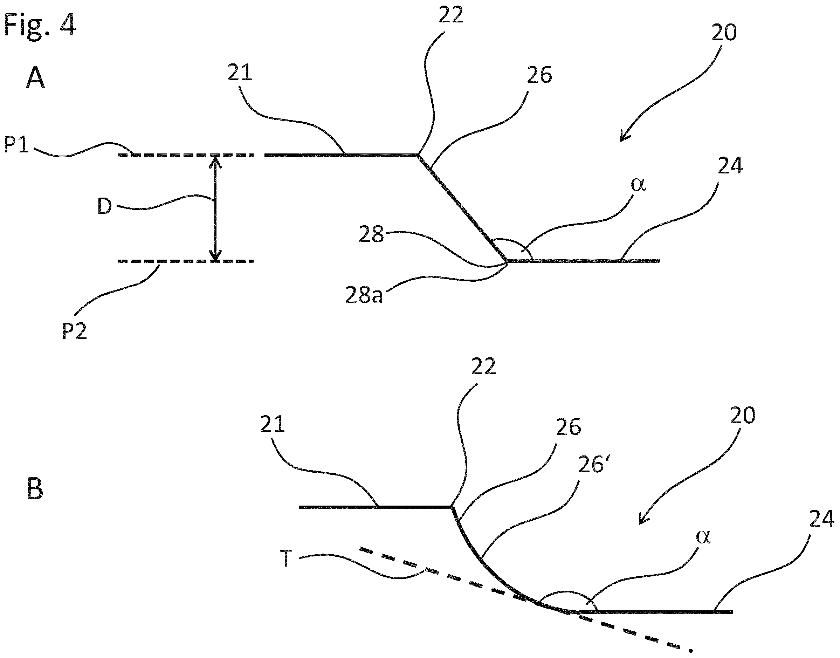

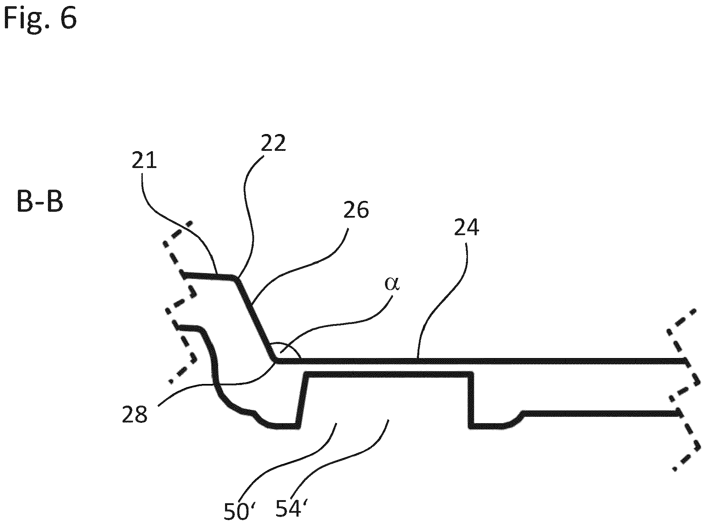

The receiving portion 20 can comprise a base section 24. The receiving portion 20 can comprise a border section 26. The receiving portion 20 can comprise an upper rim section 22. The border section 26 can connect the base section 24 and the upper rim section 22. The base section 24 can extend in a base plane P2. The upper rim section 22 can extend in a surface plane P1 (also see Figs 4A, 4B, 5, 6, 7).

The receiving portion 20 can form a slot 60 in the housing 6 of the add-on tracking device 5. In the presented embodiment, the slot 60 is formed by the support section 40 of the receiving portion 20. The slot 60 can define a first lateral wall 70 and a second lateral wall 71.

The receiving portion 20 can extend from the front face 30 of the first connector part 10 (at the front end 220 of the add-on tracking device 5) towards the foot face 32 of the first connector part 10 along a longitudinal direction L1 of the receiving portion 20.

The shape of the receiving portion 20 can be mirror-symmetric with respect to a centre axis C1 extending along the longitudinal direction L1 of the receiving portion 20. The contour of the receiving portion 20 can be mirror-symmetric with respect to a centre axis C1 extending along the longitudinal direction L1 of the receiving portion 20.

In the illustrated embodiment (Fig. 3), the receiving portion 20 comprises a support section 40, a central section 42, a first lateral section 44 and a second lateral section 46.

In the illustrated embodiment, the support section 40 is arranged at the front face 30 of the first connector part 10. Along the longitudinal direction L1 of the receiving portion 20 the central section 42 can be adjacent to the support section 40. In the illustrated embodiment, the first lateral section 44 extends lateral from the central section 42. The first lateral section 44 can extend away from the centre axis C1, particularly laterally. In the illustrated embodiment, the second lateral section 46 extends lateral from the central section 42. The second lateral section 46 can extend away from the centre axis C1, particularly laterally. In the shown embodiment, the first lateral section 44 and the second lateral section 46 extend lateral from the central section 42 in opposing directions. The first lateral section 44 and/or the second lateral section 46 can extend laterally beyond the support section 40, particularly perpendicular to the centre axis C1. The first lateral section 44 can be a convex portion. The second lateral section 46 can be a convex portion. Along the longitudinal direction L1 of the receiving portion 20 the first lateral section 44 can extend beyond the central section 42. Along the longitudinal direction L1 of the receiving portion 20 the second lateral section 46 can extend beyond the central section 42.

The upper rim section 22 can give an outline of the receiving portion 20. In the illustrated embodiment, the upper rim section 22 is continuous from the first lateral wall 70 to the second lateral wall 71. Along the extension of the upper rim section 22 from the first lateral wall 70 to the second lateral wall 71, the curvature of the upper rim section 22 in the surface plane P1 (also see Figs 4A, 4B) can change.

The outline of the first lateral section 44 can be convex. The upper rim section 22, 68 of the first lateral section 44 can be convex. The upper rim section 22, 68 of the first lateral section 44 can be curved away from the central section 42. The outline of the second lateral section 46 can be convex. The upper rim section 22, 69 of the second lateral section 46 can be convex. The upper rim section 22, 69 of the second lateral section 46 can be curved away from the central section 42.

The outline of the receiving portion 20 between the first lateral section 44 and the second lateral section 46 can be concave. The upper rim section 22 can comprise a first concave upper rim section 62 between the first lateral section 44 and the second lateral section 46. The first concave upper rim section 62 can be curved towards the central section 42. The first concave upper rim section 62 can be curved towards the slot 60. The first concave upper rim section 62 can be arranged opposite to the slot 60.

The outline of the receiving portion 20 between the first lateral section 44 and the support section 40 can be concave. The upper rim section 22 can comprise a second concave upper rim section 64 between the first lateral section 44 and the support section 40. The second concave upper rim section 64 can be curved towards the centre axis C1.

The outline of the receiving portion 20 between the second lateral section 46 and the support section 40 can be concave. The upper rim portion 22 can comprise a third concave upper rim section 66 between the second lateral section 46 and the support section 40. The third concave upper rim section 66 can be curved towards the centre axis C1.

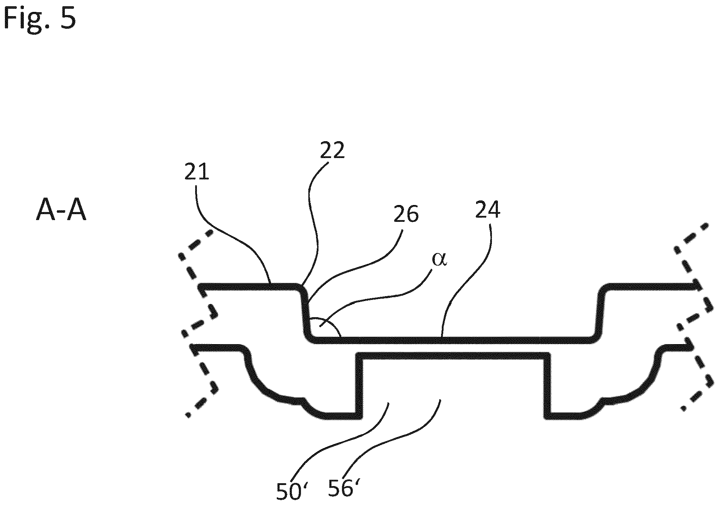

The illustrated embodiment, the first connector part 10 comprises three magnetic elements 50, 52, 54, 56. In the example given in Fig. 3, the first magnetic element 52 is arranged in the first lateral section 44, the second magnetic element 54 is arranged in the second lateral section 46 and the third magnetic element 56 is arranged in the support section 40. The three magnetic elements 52, 54, 56 can be spaced apart from each other. The magnetic elements 50, 52, 54, 56 can be arranged in the base section 24. The magnetic elements 50, 52, 54, 56 can be arranged adjacent to the base section 24. In the embodiments shown in Figs 5, 6, and 7, receptacles 50', 54', 56' are illustrated for receiving the respective magnetic elements 50, 54, 56.

The border section 26 can extend from the upper rim section 22 to a lower rim section 28a. The receiving portion 20 can comprise a contact line 28 between the border section 26 and the base section 24 (particularly, see Figs 3, 4A, 4B). In an embodiment, the lower rim section 28a can represent the contact line 28 (particularly, see Fig. 4A). In the embodiment illustrated in Fig. 3, the contact line 28 is continuous from the first lateral wall 70 to the second lateral wall 71. Along the extension of the contact line 28 from the first lateral wall 70 to the second lateral wall 71, the curvature of contact line 28 in the base plane P2 (also see Figs 4A, 4B) can change.

The border section 26 and the base section 24 can enclose an obtuse angle α (particularly see Figs 4A, 4B, 5, 6, 7). In the embodiment illustrated in Fig. 4B, the border section 26, 26' is curved. A tangent T of the curved portion 26' and the base section 24 can enclose the obtuse angle α.

In different sections of the receiving portion 20, the obtuse angle α can have different values, particularly in different sections along the circumference of the receiving portion 20 (Figs 3, 5, 6, 7). In the illustrated example, in a section of the receiving portion 20 showing a convex outline (or upper rim section 22) the obtuse angle α is larger than in a section of the receiving portion 20 showing a concave outline (or upper rim section 22).

In the embodiment shown in Figs 3, 5, 6, 7, the obtuse angle α is larger in the first lateral section 44 than in the support section 40. In the embodiment shown in Figs 3, 5, 6, 7, the obtuse angle α is larger in the second lateral section 46 than in the support section 40. The obtuse angle α in the first lateral section 44 can be larger than in the section between the first lateral section 44 and the second lateral section 46. The obtuse angle α in the second lateral section 44 can be larger than in the section between the first lateral section 44 and the second lateral section 46 (particularly see Figs 5, 6, 7).

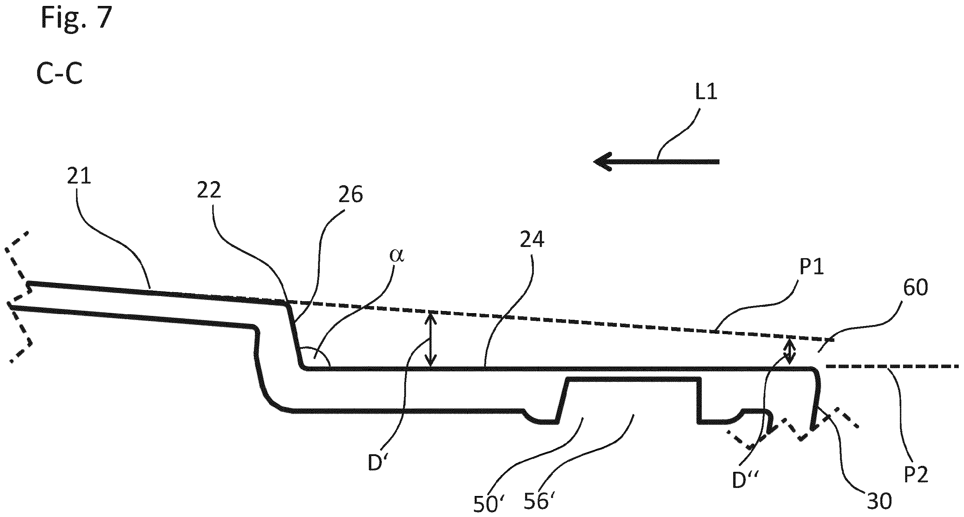

In the illustrated example, the depth D, D', D" of the receiving portion 20 increases from the front face 30 towards the foot face 32. The depth D can be the distance between the surface plane P1 and the base plane P2 perpendicular to the base plane P2 (see particularly Figs 4A, 7). The base plane P2 can extend angled with respect to the surface plane P1 (Fig. 7). The base section 24 may have a slope and rise towards the front face 30. Perpendicular to the base plane P2, the distance between the surface plane P1 and the base plane P2 can increase from the front face 30 towards to foot face 32.

An embodiment of the second connector part 110 is illustrated in Figs 8, 9 and 10. The second connector part 110 can comprise a device connector portion 112. The second connector part 110 can comprise a bridging portion 114. The second connector part 110 can comprise the inserter portion 120. The bridging portion 114 can link the device connector portion 112 and the inserter portion 120.

The device connector portion 112 can comprise a circumferential wall 171 delimiting a through-opening 170. The device connector portion 112 can be configured such that the front section 202 of a medical imaging device 3 can be inserted in the through-opening 170, particularly such that the circumferential wall 171 embraces the medical imaging device 3 and the tip 203 of the front section 202 protrudes beyond the circumferential wall 171 (also see Fig. 1).

The bridging portion 114 can extend from the device connector portion 112, particularly from the circumferential wall 171. The bridging portion 114 can extend such from the circumferential wall 171 that, when the second connector part 110 is arranged at the medical imaging device 3, the inserter portion 120 is located more distant to the front portion 202 of the medical imaging device 3 than the device connector portion 112 (also see Fig. 1). The bridging portion 114 can extend angled to a circumferential direction U of the circumferential wall 171. In an embodiment, the bridging portion 114 can extend such that the inserter portion 120 protrudes at least partially radially beyond the circumferential wall 171. The bridging portion 114 can extend such from the circumferential wall 171 that, when the second connector part 110 is arranged at the medical imaging device 3, the inserter portion 120 is located at a distance from the housing 4 of the medical imaging device 3 (also see Fig. 1). The bridging portion 114 can taper from the device connector portion 112 towards the inserter portion 120.

The inserter portion 120 can comprise a top section 122. The inserter portion 120 can comprise a bottom section 124. The inserter portion 120 can comprise an edge section 126. The edge section 126 can connect the top section 122 and the bottom section 124. The bottom section 124 can extend in a bottom plane P4. The top section 122 can extend in a top plane P3. The bottom section 124 can extend parallel to the top section 122. In particular, the bottom plane P4 can extend parallel to the top plane P3. In the illustrated embodiment, the bottom plane P4 and the top plane P3 extend angled to the circumferential direction U of the circumferential wall 171. In an embodiment, the bottom plane P4 (bottom section 124) and the device connector portion 112 enclose an obtuse angle.

In the illustrated embodiment, the inserter portion 120 is configured and arranged such with respect to the device connector portion 112 that the bottom section 124 faces away from the device connector portion 112. The spacer 116 can be arranged at the top section 122, facing towards the device connector portion 112.

The inserter portion 120 can extend from the bridging portion 114 towards a tip 128 along a longitudinal direction L2 of the inserter portion 120.

The shape of the inserter portion 120 can be mirror-symmetric with respect to a centre axis C2 of the inserter portion 120 extending along the longitudinal direction L2 of the inserter portion 120. The contour of the inserter portion 120 can be mirror-symmetric with respect to a centre axis C2 extending along the longitudinal direction L2 of the inserter portion 120.

In the illustrated embodiment (Figs 8, 9, 10), the inserter portion 120 comprises a support section 140, a central section 142, a first lateral section 144 and a second lateral section 146.

In the illustrated embodiment, the support section 140 is arranged adjacent to the bridging section 114 of the second connector part 110. Along the longitudinal direction L2 of the inserter portion 120 the central section 142 can be adjacent to the support section 140. In the illustrated embodiment, the first lateral section 144 extends lateral from the central section 142. The first lateral section 144 can extend away from the centre axis C2, particularly laterally. In the illustrated embodiment, the second lateral section 146 extends lateral from the central section 142. The second lateral section 146 can extend away from the centre axis C2, particularly laterally. In the shown embodiment, the first lateral section 144 and the second lateral section 146 extend lateral from the central section 142 in opposing directions. The first lateral section 144 and/or the second lateral section 146 can extend laterally beyond the support section 140, particularly perpendicular to the centre axis C2. The first lateral section 144 can be a convex portion. The second lateral section 146 can be a convex portion. Along the longitudinal direction L2 of the inserter portion 120 the first lateral section 144 can extend beyond the central section 142. Along the longitudinal direction L2 of the inserter portion 120 the second lateral section 146 can extend beyond the central section 142.

The inserter portion 120 can comprise a contour. Along the extension of the contour, the curvature of the contour can change. The contour can extend from the support section 140 along the first lateral section 144 and the second lateral section 146 back to the support section 140. The edge section 126 can define the contour.

The first lateral section 144 of the inserter portion 120 can have a convex contour section 168. The second lateral section 146 of the inserter portion 120 can have a convex contour section 169. The contour between the first lateral section 144 and the second lateral section 146 can be the first concave contour section 162. The contour between the first lateral section 144 and the second lateral section 146 can be curved towards the central section 142. The inserter portion 120 can comprise a second concave contour section 164. The second concave contour section 164 can be arranged between the first lateral section 144 and the support section 140. The inserter portion 120 can comprise a third concave contour section 166. The third concave contour section 166 can be arranged between the second lateral section 146 and the support section. The second concave contour section 144 and/or the third concave contour section can be curved towards the centre axis C2.

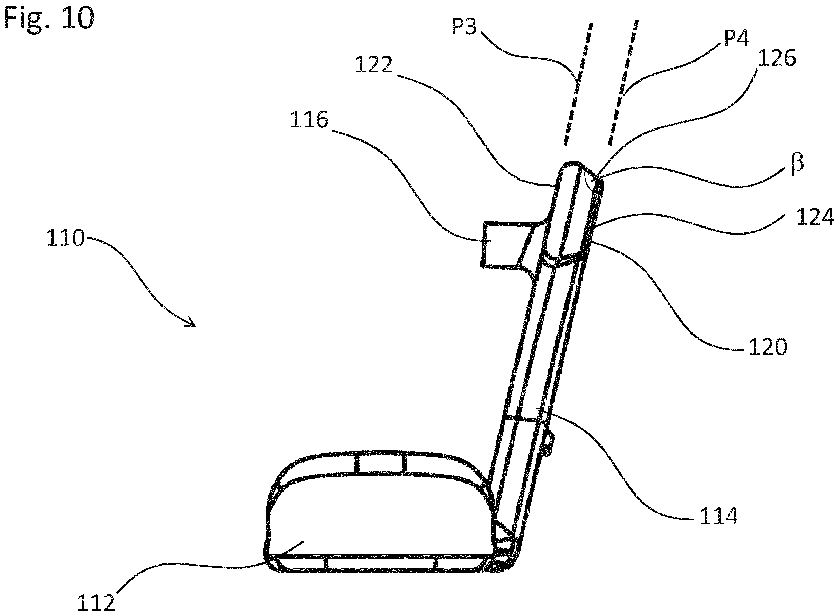

The edge section 126 and the bottom section 124 can enclose an obtuse angle β (particularly see Fig. 10).

In different sections of the inserter portion 120, the obtuse angle β can have different values, particularly in different sections along the circumference of the inserter portion 120 (see particularly Fig. 8). In the illustrated example, in a section of the inserter portion 120 showing a convex contour the obtuse angle β is larger than in a section of the inserter portion 120 showing a concave contour.

In the embodiment shown in Figs 8, 9, 10, the obtuse angle β is larger in the first lateral section 144 than in the support section 140. In the illustrated embodiment, the obtuse angle β is larger in the second lateral section 146 than in the support section 140. The obtuse angle β in the first lateral section 144 can be larger than in the section between the first lateral section 144 and the second lateral section 146. The obtuse angle β in the second lateral section 146 can be larger than in the section between the first lateral section 144 and the second lateral section 146.

The second connector part 110 can comprise three magnetic elements 152, 154, 156 (see Fig. 2). The first magnetic element 152 can be arranged in the first lateral section 142. The second magnetic element 154 can be arranged in the second lateral section 144. The third magnetic element 156 can be arranged in the support section 140. The three magnetic elements 152, 154, 156 can be spaced apart from each other.

## Claims

1. A connector system (1) for connecting an add-on unit (5) and a hand-held medical imaging device (3),
wherein the connector system (1) comprises an add-on unit-side first connector part (10) comprising a recessed receiving portion (20), and
a medical-imaging-device-side second connector part (110) comprising a protruding inserter portion (120),
wherein the receiving portion (20) comprises an upper rim section (22), a base section (24) and a border section (26) connecting the upper rim section (22) and the base section (24),
wherein the border section (26) and the base section (24) enclose an obtuse angle (α),
wherein the inserter portion (120) comprises a bottom section (124) and an edge section (126) connected to the bottom section (124),
wherein the edge section (126) is form-fit to the border section (26) such that the inserter portion (120) is receivable by the receiving portion (20) in a unique pre-determined orientation with respect to each other,
wherein the receiving portion (20) and the inserter portion (120) each comprises a magnetic element (50) configured to provide a magnetic connection between each other to fixate the inserter portion (120) in the receiving portion (20) in the pre-determined orientation.

2. The connector system (1) according to claim 1, wherein the receiving portion (20) extends from a front face (30) of the first connector part (10) towards an opposing foot face (32) of the first connector part (10), wherein a depth (D, D', D") of the receiving portion (20) increases from the front face (30) towards the foot face (32).

3. The connector system (1) according to claim 1 or 2, wherein the receiving portion (20) comprises a first magnetic element (52) of the receiving portion (20) and a spatially separated second magnetic element (54) of the receiving portion (20), wherein a polarity of the first magnetic element (52) of the receiving portion (20) is opposite to a polarity of the second magnetic element (54) of the receiving portion (20),
and/or
wherein the inserter portion (120) comprises a first magnetic element (152) of the inserter portion (120) and a spatially separated second magnetic element (154) of the inserter portion (120), wherein a polarity of the first magnetic element (152) of the inserter portion (120) is opposite to a polarity of the second magnetic element (154) of the inserter portion (120),
particularly
wherein the inserter portion and the receiving portion are configured such that the first magnetic element (152) of the inserter portion (120) aligns with the first magnetic element (52) of the receiving portion (20), when the inserter portion (120) is received by the receiving portion (20) in the pre-determined orientation, wherein the polarity of the first magnetic element (152) of the inserter portion (120) is opposite to the polarity of the first magnetic element (52) of the receiving portion (20), and/or wherein the inserter portion and the receiving portion are configured such that the second magnetic element (154) of the inserter portion (120) aligns with the second magnetic element (54) of the receiving portion (20), when the inserter portion (120) is received by the receiving portion (20) in the pre-determined orientation, wherein the polarity of the second magnetic element (154) of the inserter portion (120) is opposite to the polarity of the second magnetic element (54) of the receiving portion (20).

4. The connector system (1) according to one of the claims 1 to 3, wherein the receiving portion (20) extends from a front face (30) of the first connector part (10) towards an opposing foot face (32) of the first connector part (10) along a longitudinal direction (L1) of the receiving portion (20), wherein the shape of the receiving portion (20) is reflection symmetric with respect to a centre axis (C1) of the receiving portion (20) extending along the longitudinal direction (L1) of the receiving portion (20),
and/or
wherein the inserter portion (120) extents from a bridging section (114) of the second connector part (110) towards an opposing tip (128) along a longitudinal direction (L2) of the inserter portion (120), wherein the shape of the inserter portion (120) is reflection symmetric with respect to a centre axis (C2) of the inserter portion (120) extending along the longitudinal direction (L2) of the inserter portion (120).

5. The connector system (1) according to one of the claims 1 to 4, wherein the receiving portion (20) comprises a support section (40) of the receiving portion (20), a central section (42) of the receiving portion (20), a first lateral section (44) of the receiving portion (20) and a second lateral section (46) of the receiving portion (20), wherein the first lateral section (44) of the receiving portion (20) and the second lateral section (46) of the receiving portion (20) protrude laterally beyond the central section (42) of the receiving portion (20), wherein the border section (26) and the base section (24) enclose a larger obtuse angle (α) in the first lateral section (44) of the receiving portion (20) and/or in the second lateral section (46) of the receiving portion (20) than in the support section (40) of the receiving portion (20),
and/or
wherein the inserter portion (120) comprises a support section (140) of the inserter portion (120), a central section (142) of the inserter portion (120), a first lateral section (144) of the inserter portion (120) and a second lateral section (146) of the inserter portion (120), wherein the first lateral section (144) of the inserter portion (120) and the second lateral section (146) of the inserter portion (120) protrude laterally beyond the central section (142) of the inserter portion (120), wherein the edge section (126) and the bottom section (124) enclose a larger obtuse angle (β) in the first lateral section (144) of the inserter portion (120) and/or in the second lateral section (146) of the inserter portion (120) than in the support section (140) of the inserter portion (120).

6. The connector system (1) according to one of the claims 1 to 5,
wherein the receiving portion (20) comprises a support section (40), a central section (42), a first lateral section (44) and a second lateral section (46), wherein the first lateral section (44) and the second lateral section (46) protrude laterally beyond the central section (42),
wherein the receiving portion (20) has a first concave upper rim section (62) between the first lateral section (44) of the receiving portion (20) and the second lateral section (46) of the receiving portion (20), and/or
wherein the receiving portion (20) has a second concave upper rim section (64) between the first lateral section (44) of the receiving portion (20) and the support section (40) of the receiving portion (20) and a third concave upper rim section (66) between the second lateral section (46) of the receiving portion (20) and the support section (40) of the receiving portion (20), and/or
wherein the first lateral section (44) of the receiving portion (20) has a convex upper rim section (62, 68), and/or
wherein the second lateral section (46) of the receiving portion (20) has a convex upper rim section (62, 69).

7. The connector system (1) according to one of the claims 1 to 6,
wherein the inserter portion (120) comprises a support section (140), a central section (142), a first lateral section (144) and a second lateral section (146), wherein the first lateral section (144) and the second lateral section (146) protrude laterally beyond the central section (142),
wherein the inserter portion (120) has a first concave contour section (162) between the first lateral section (144) of the inserter portion (120) and the second lateral section (146) of the inserter portion (120), and/or
wherein the inserter portion (120) has a second concave contour section (164) between the first lateral section (144) of the inserter portion (120) and the support section (140) of the inserter portion (120) and a third concave contour section (166) between the second lateral section (146) of the inserter portion (120) and the support section (140) of the inserter portion (120), and/or
wherein the first lateral section (144) of the inserter portion (120) has a convex contour section (168), and/or
wherein the second lateral section (146) of the inserter portion (120) has a convex contour section (169).

8. The connector system (1) according to one of the claims 1 to 7,
wherein the receiving portion (20) forms a slot (60) in a front face (30) of the first connector part (10), particularly wherein the support section (40) of the receiving portion (20) forms the slot (60) in the front face (30) of the first connector part (10), particularly in a housing (6) of the add-on unit (5),
particularly wherein a support section (140) of the inserter portion (120) is configured to be at least partially insertable in the slot (60) of the first connector part (10).

9. The connector system (1) according to one of the claims 1 to 8, wherein the second connector part (110) comprises a bridging section (114) connecting a device connector portion (112) and the inserter portion (120),
wherein the device connector portion (112) is configured for repeatedly releasably connecting the second connector part (110) and the hand-held medical imaging device (3), and/or
wherein the bottom section (124) faces away from the device connector portion (112),
and/or wherein the bottom section (124) and the device connector portion (112) enclose an obtuse angle,
and/or wherein the second connector part (110) comprises a spacer (116), wherein the spacer is arranged and configured such that the spacer (116) points away from the bottom section (124), particularly wherein the spacer (116) points towards the device connector portion (112), particularly wherein the spacer (116) is arranged and configured such that the spacer (116) abuts a housing (4) of the hand-held medical imaging device (3), when the second connector part (110) is arranged at the hand-held medical imaging device (3), particularly wherein the spacer (116) is arranged at the top section (122).

10. The connector system (1) according to one of the claims 1 to 9,
wherein the add-on unit (5) is an add-on tracking device (5) or an add-on tracking target.

11. The connector system (1) according to one of the claims 1 to 10,
wherein the hand-held medical imaging device (3) is a hand-held ultrasonic device.

12. A method for connecting an add-on unit (5), particularly an add-on tracking device (5), and a hand-held medical imaging device (3) using a connector system (1) for connecting the add-on unit (5) and the hand-held medical imaging device (3) according to one of the claims 1 to 11, wherein the connector system (1) is connected with the hand-held medical imaging device (3), particularly via the device connector portion (112), the method comprising:
- an insertion step, wherein in the insertion step the inserter portion (120) of the medical-imaging-device-side second connector part (110) is inserted in the receiving portion (20) of the add-on unit-side first connector part (10) in the pre-determined orientation,
- a fixation step, wherein in the fixation step, the magnetic connection between the inserter portion (120) and the receiving portion (20) in the pre-determined orientation is provided by the magnetic elements (50) of the inserter portion (120) and the receiving portion (20).

13. An add-on-unit-side first connector part (10) for the connector system (1) according to one of the claims 1 to 11 for connecting the add-on unit (5) and the hand-held medical imaging device (3),
wherein the add-on-unit-side first connector part (10) comprises the recessed receiving portion (20), wherein the receiving portion (20) is configured for receiving the protruding inserter portion (120) of the medical-imaging-device-side second connector part (110) in the unique pre-determined orientation,
wherein the receiving portion (20) comprises the upper rim section (22), the base section (24) and the border section (26) connecting the upper rim section (22) and the base section (24),
wherein the border section (26) and the base section (24) enclose the obtuse angle,
wherein the border section (26) is arranged and configured such that the border section (26) is form-fit to the edge section (126) of the inserter portion (120) such that the inserter portion (120) is receivable by the receiving portion (20) in the unique pre-determined orientation with respect to each other,
wherein the receiving portion (20) comprises the magnetic element (50) configured to provide the magnetic connection with the inserter portion (120) to fixate the inserter portion (120) in the receiving portion (20) in the pre-determined orientation.

14. A medical-imaging-device-side second connector part (110) for the connector system (1) according to one of the claims 1 to 11 for connecting the add-on unit (5) and the hand-held medical imaging device (3),
wherein the medical-imaging-device-side second connector part (110) comprises the protruding inserter portion (120), wherein the inserter portion (120) is configured for insertion in the recessed receiving portion (20) of the add-on-unit-side first connector part (10) in the unique pre-determined orientation,
wherein the inserter portion (120) comprises the bottom section (124) and the edge section (126) connected to the bottom section (124),
wherein the edge section (126) is arranged and configured such that the edge section (126) is form-fit to the border section (26) of the receiving portion (20) such that the inserter portion (120) is receivable by the receiving portion (20) in the unique pre-determined orientation with respect to each other,
wherein the inserter portion (120) comprises the magnetic element (50) configured to provide the magnetic connection with the receiving portion (20) to fixate the inserter portion (120) in the receiving portion (20) in the pre-determined orientation.

15. A medical system (8) comprising a hand-held medical imaging device (3), an add-on unit (5), and a connector system (1) for connecting the add-on unit (5) and the hand-held medical imaging device (3), particularly the connector system (1) according to one of the claims 1 to 11,
wherein the connector system (1) comprises a add-on-unit-side first connector part (10) comprising a recessed receiving portion (20), and
a medical-imaging-device-side second connector part (110) comprising a protruding inserter portion (120),
wherein the receiving portion (20) comprises an upper rim section (22), a base section (24) and a border section (26) connecting the upper rim section (22) and the base section (24),
wherein the border section (26) and the base section (24) enclose an obtuse angle,
wherein the inserter portion (120) comprises a bottom section (124) and an edge section (126) connected to the bottom section (124),
wherein the edge section (126) is form-fit to the border section (26) such that the inserter portion (120) is receivable by the receiving portion (20) in a unique pre-determined orientation with respect to each other,
wherein the receiving portion (20) and the inserter portion (120) each comprises a magnetic element (50) configured to provide a magnetic connection between each other to fixate the inserter portion (120) in the receiving portion (20) in the pre-determined orientation,
particularly wherein the hand-held medical imaging device (3) is an ultrasonic device, and/or
particularly, wherein the add-on unit is an add-on tracking device (5).
